# EUROPEAN PATENT APPLICATION

(11) **EP 2 520 936 A1**
(43) Date of publication of application: **07.11.2012**
(21) Application number: 12168350.2
(22) Date of filing: 13.10.2006
(51) Int. Cl.: G01N 33/68

(54) **Diagnosis and monitoring of chronic renal disease using NGAL**

(30) Priority: 13.10.2005 US 374285
(62) Divisional of application: 06816888.9
(71) Applicant: Children's Hospital Medical Center, Cincinnati, Ohio 45229 (US); The Trustees of Columbia University, New York NY 10027 (US)
(72) Inventor: Barasch, Jonathan, M., New York City, NY New York 10025 (US); Devarajan, Prasad, Cincinnati, OH Ohio 45242 (US); Nickolas, Thomas, L., Brooklyn, NY New York 11201 (US); Mori, Kiyoshi, Kyoto, 603-8179 (JP)
(74) Representative: Huenges, Martin

(57) **Abstract**

The present invention relates generally to the area of assays for NGAL. In particular, the invention relates to assays using NGAL to monitor and assess chronic renal disease, and including methods, kits for the assay, and kit components.

## Description

### FIELD OF THE INVENTION

The present invention relates generally to the area of assays for NGAL. In particular, the invention relates to assays using NGAL to monitor and assess chronic renal disease, and including methods, kits for the assay, and kit components.

### BACKGROUND OF THE INVENTION

Over the past twenty years it has been learned that earlier identification and treatment of kidney disease can prevent kidney disease progression. Thus, a biomarker of kidney damage that indicates the presence of early damage and can be used to identify patients at an increased risk of progressive disease would favorably impact kidney disease diagnosis and treatment. Serum creatinine, the current marker of kidney function, is influenced by muscle mass, gender, race, and medications. In addition, repetitive measurements of creatinine are required to diagnose progressive renal failure. These limitations often result in the diagnosis of kidney disease only after significant damage has already occurred. Higher degrees of damage at diagnosis limit the efficacy of kidney function preservation therapies and result in higher disease progression rates. Our armamentarium against kidney disease relies upon early intervention and includes interrupting the renin-angiotensin system, and aggressive blood pressure, diabetes, and lipid control.

An early marker of kidney damage would promote earlier intervention in order to arrest the progression to end-stage renal disease (ESRD), the condition where the kidneys permanently fail to work. In order to be of use to the general clinician, the biomarker preferably indicates renal damage prior to and earlier than the current indicators of kidney function, be available non-invasively, and be easily interpretable without the use of complex corrections, and only require a single measurement.

The practical impact of an early marker of kidney disease is best demonstrated by reviewing the changing demographics of kidney disease. The worldwide epidemic of chronic renal disease (CRD) will double the incidence of end-stage renal disease over the next decade, and have a direct impact on healthcare expenditures. But this only represents the tip of the iceberg since the number of patients with earlier stages of chronic renal disease is estimated to exceed those reaching end-stage renal disease by more than 50 times. Early identification of chronic renal disease and timely detection of progression are truly global challenges facing the nephrology community, especially since a number of promising primary and secondary interventions to decelerate the progression are available. In order to control costs, physicians will need to decrease progression rates of chronic renal disease to end-stage renal disease. Even small decreases in progression rates can result in large economic gains if patients are prevented from requiring renal replacement therapy (RRT).

The current markers of kidney disease and kidney disease progression are the serum creatinine and urinary protein concentration, including microalbuminuria. The slope of the decrease in glomerular filtration rate (GFR) has been demonstrated to predict the timing of ESRD, and the level of proteinuria has been shown in multiple studies to correlate with kidney disease progression rates. These are useful biomarkers of kidney disease and its progression that have withstood the scrutiny of multiple studies. However, their ability to recognize early kidney disease is limited. Serum creatinine concentration is recognized as an unreliable measure of kidney function because it is dependent on the subject's age, gender, race, muscle mass, weight, degree of physical exertion, and various medications. Correct interpretation of kidney function based on serum creatinine requires complex formulas that are not routinely employed by practicing medical providers. In addition, an understanding of whether the disease is progressive requires serial creatinine measurements. Although urinary protein is very sensitive for progressive renal disease, its appearance occurs after significant renal damage has already occurred. For maximum usefulness, a biomarker of early and/or progressive kidney damage should become positive at the earliest point that kidney damage begins to occur.

Thus, there is an active search for kidney biomarkers that can predict a patient's risk of progressive chronic renal disease, with the hope that early identification of kidney disease will lead to early treatment, or that the biomarker will identify a treatable entity that can depress rates of kidney disease progression. Some examples of promising kidney biomarkers include asymmetric dimethylarginine (ADMA), liver-type fatty acid-binding protein (L-FABP), cystatin C, C-reactive Protein (CRP), and soluble tumor necrosis factor receptor II (sTNFrii). It is not yet clear how these biomarkers will affect chronic renal disease treatment, how effective they are at detecting the extent of kidney damage, and whether they are even feasible for widespread clinical use. It is also not clear how the appearance of these markers correlates, if at all, with the markers serum creatinine and proteinuria. In fact, none of these biomarkers are known to provide a direct measure of kidney damage.

Cystatin C and L-FABP are produced by cells outside the kidney and rely upon filtration across the glomerulus. ADMA is an endogenous nitric oxide synthase (NOS) inhibitor. Elevated levels have been shown to predict kidney disease progression rates. CRP and sTNFrii are measures of inflammatory activity. Their levels have been shown to correlate with kidney disease progression in inflamed states. CRP appears to correlate with endothelial injury, while sTNFrii has been associated with glomerular injury. Out of these biomarkers, only ADMA, CRP, and sTNFrii might represent guides to therapy. However, there is no published literature on their ability to detect preclinical kidney disease.

Other potential biomarkers include kidney extracellular matrix probes. Previous studies have demonstrated that the degree of tubulointerstitial (TI) alterations at renal biopsy are highly correlated with renal function and prognosis. These alterations result from the deposition of extracellular matrix (ECM) molecules in response to renal injury. The use of ECM probes and ECM-related (ECMR) probes to assess renal outcomes has recently been reviewed. Although ECM and ECMR probes appear promising in their ability to predict the development of microalbuminuria, and progression of renal disease, they are not easily employed because such tests require a kidney biopsy.

Adverse outcomes to kidney disease are based on the level of kidney function and risk of loss of function in the future. Chronic kidney disease tends to worsen over time. Therefore, the risk of adverse outcomes increases over time with disease severity. Many disciplines in medicine, including related specialties of hypertension, cardiovascular disease, diabetes, and transplantation, have adopted classification systems based on severity, to guide clinical interventions, research, and professional and public education. Such a model is essential for any public health approach to this disease.

The ability to slow and arrest the progression of chronic renal disease has been a paradigm shift in nephrology. Multiple studies have demonstrated that tight blood pressure and glycemic control, and the use of agents that block the renin-angiotensin system can decrease the rate of decline in kidney function. Earlier and more aggressive treatment of diabetes, hypertension, and proteinuria has been the most effective method to prevent the development and progression of chronic kidney disease. While the recognition and modification of these risk factors has been invaluable, large clinical studies have noted that the incidence and progression of chronic renal disease is dangerously increasing and can vary substantially among the population at risk for kidney disease. Therefore, further improvement in prevention and treatment recommendations must promote earlier identification of patients at a higher risk of disease progression.

Recent guidelines from the National Kidney Foundation (NKF) and the National Institute of Diabetes and Digestive Diseases (NIDDK) have called for the identification of new markers of kidney damage. Identification of new markers of risk stratification may result from both biochemical assays as well as from human genetics. Thus, there clearly remains a need for additional methods and biomarkers for the early detection of chronic renal disease.

### SUMMARY OF THE INVENTION

The present invention provides among other things methods of assessing the present and ongoing kidney status in a mammalian subject afflicted with or at a risk of developing chronic renal disease (CRD) and/or chronic renal failure (CRF), and with worsening CRD and CRF, by detecting the quantity (e.g., determining the level) of Neutrophil Gelatinase-Associated Lipocalin (NGAL) in a body fluid sample. The invention also provides a method of monitoring the effectiveness of a treatment for chronic renal injury by determining the level of NGAL in the body fluid before and in particular after the treatment. The properties and characteristics of NGAL as a biomarker allow for its use in this manner for the early detection of chronic renal injury or changes in chronic renal injury status.

One aspect of the invention provides a method for the early detection of a chronic renal injury in a mammal, comprising the steps of: i) obtaining or providing a sample of a body fluid from a mammal that is not experiencing an acute renal injury, the body fluid selected from the group consisting of urine, plasma, and serum; ii) detecting (e.g., determining) the level of NGAL in the sample (e.g., using an antibody against NGAL); and iii) evaluating the chronic renal injury status of the subject, based on the level of NGAL in the sample. The evaluation of the chronic renal injury status can be used to determine whether the chronic renal injury is sub-clinical, stable, or progressing (progressive renal disease). The method also provides an evaluation of the renal status as a progressive or worsening renal injury with only a single sampling and assay.

Another aspect of the invention provides a method for the detection of any change in a chronic renal injury status of a mammal, comprising the steps of: i) obtaining a first sample of a body fluid from a mammal that is not experiencing an acute renal injury, the body fluid selected from the group consisting of urine, plasma, and serum; ii) detecting (e.g., determining) the level of NGAL in the first sample (e.g., using an antibody against NGAL); iii) obtaining at least one subsequent sample of the body fluid from the mammal after a period of time after obtaining the first sample; iv) detecting (e.g., determining) the level of NGAL in the at least one subsequent sample (e.g., using an antibody against NGAL); and v) evaluating the chronic renal injury status of the mammal, based on comparing the level of NGAL in the at least one subsequent sample to the level of NGAL in the first sample. Generally, a higher level of NGAL in the subsequent sample is an indication of a declining or worsening chronic renal injury status in the subject (e.g., and potentially of a worsening chronic renal injury), and a reduced level of NGAL in the subsequent sample is an indication of an improving chronic renal injury status in the subject (e.g., and potentially of an improving chronic renal injury).

Another aspect of the invention provides a method of monitoring the effectiveness of a treatment for chronic renal injury in a mammal, comprising the steps of: i) providing or obtaining a baseline sample of a body fluid from a mammal experiencing a chronic renal injury, the body fluid selected from the group consisting of urine, plasma, and serum; ii) detecting (e.g., determining) the level of NGAL in the baseline sample (e.g., using an antibody against NGAL); iii) providing at least one treatment for the chronic renal injury to the mammal; iv) providing or obtaining at least one post-treatment sample of the body fluid from the mammal; v) detecting (e.g., determining) the level of NGAL in the post-treatment sample (e.g., using an antibody against NGAL); and vi) evaluating the effectiveness of the treatment, based on comparing the level of NGAL in the post-treatment sample to the level of NGAL in the baseline sample.

A further aspect of the invention provides a method of identifying the extent of chronic renal injury in a mammal over time, comprising the steps of: i) obtaining at least one first sample of a body fluid at a first time from a mammal that is not experiencing an acute renal injury, the body fluid selected from the group consisting of urine, plasma, and serum; ii) detecting (e.g., determining) the level of NGAL in the first sample (e.g., using an antibody against NGAL); iii) obtaining at least one subsequent sample of the body fluid at a time subsequent to the first time, from the mammal; iv) detecting (e.g., determining) the level of NGAL in the at least one subsequent sample (e.g., using an antibody against NGAL); and v) determining the extent of the chronic renal injury in the mammal over time, based on comparing the level of NGAL in the at least one subsequent sample to the level of NGAL in the first sample.

Typically the mammalian subject is a human. Where more than one subsequent sample is drawn, they are typically obtained and provided intermittently from the subject, and at predetermined times, ranging from one or more days, to one or more weeks, to one or more months, to one or more years. Other sampling regimens also can be employed.

Typically the mammalian subject is also evaluated to determine if the subject is experiencing another condition that may contribute to the level of NGAL in the sample, such condition including, but limited to, an acute bacterial or viral infection, acute inflammation, an acute or chronic injury to another organ, and a cancer. Such another condition typically does not effect or cause an injury to the kidney. However, such condition on its own can contribute an amount of NGAL into the blood stream, and in some cases into the urine, making it difficult to distinguish such NGAL from NGAL that is expressed as a direct result of a chronic renal injury. Some types of other conditions can effect high levels of NGAL that can overwhelm the concentration of NGAL resulting from the chronic renal injury.

### BRIEF DESCRIPTION OF THE DRAWINGS

FIG. 1 shows mean urinary NGAL levels by etiology of CRD patients.

FIG. 2 shows the logarithm (log) of NGAL and serum creatinine in patients that progressed to endpoint.

FIG. 3 shows the log of NGAL and serum creatinine in patients that did not progress to endpoint.

FIG. 4 shows the log of NGAL and urine protein to creatinine ratio in patients that progressed to endpoint.

FIG. 5 shows the log of NGAL and urine protein to creatinine ratio in patients that did not progress to endpoint.

FIG. 6 shows a Kaplan-Meier Curve for Urinary NGAL.

FIG. 7 shows a Kaplan-Meier Curve for Urinary Protein.

FIG. 8 shows the association between urinary NGAL and percent interstitial fibrosis in kidney biopsy.

FIG. 9 shows the correlation of levels of serum NGAL and cystatin C levels in a population of CRD patients.

FIG. 10A shows the correlation of cystatin C with serum creatinine in the population of CRD patients.

FIG. 10B shows the correlation of cystatin C with eGFR in the population of CRD patients.

FIG. 10C shows the correlation of natural logarithm (In) NGAL with serum creatinine in the population of CRD patients.

FIG. lOD shows the correlation of In NGAL with eGFR in the population of CRD patients.

FIG. 11A shows the correlation of cystatin C with measured GFR in the population of CRD patients.

FIG. 11B shows the correlation of In NGAL with measured GFR in the population of CRD patients.

FIG. 11C shows the correlation of eGFR with measured GFR in the population of CRD patients.

FIG. 12A shows the Receiver Operating Characteristics (ROC) analyses for serum cystatin C for a GFR cut-off point of 60 mL/min/1.73m².

FIG. 12B shows the ROC analyses for serum NGAL for a GFR cut-off point of 60 mL/min/1.73m².

FIG. 12C shows the ROC analyses for eGFR for a GFR cut-off point of 60 mL/min/1.73m²_{.}

FIG. 13A shows the Receiver Operating Characteristics (ROC) analyses for serum cystatin C for a GFR cut-off point of 30 mL/min/1.73m².

FIG. 13B shows the ROC analyses for serum NGAL for a GFR cut-off point of 30 mL/min/1.73m².

FIG. 13C shows the ROC analyses for eGFR for a GFR cut-off point of 30 mL/min/1.731m².

### DETAILED DESCRIPTION OF THE INVENTION

### Definition:

Unless defined otherwise, all technical and scientific terms used herein have the same meaning as commonly understood by one of ordinary skill in the art to which this invention belongs.

As used herein, the phrases "chronic renal tubular cell injury", "progressive renal disease", "chronic renal failure" (or CRF), "chronic renal disease" (or CRD), "chronic kidney disease" (or CKD), "chronic kidney injury", as well as other synonymous phrases, are all "chronic renal injury". Chronic renal injury includes any kidney condition, dysfunction or injury that: (a) occurs over a prolonged or gradual period of time (e.g., minimally weeks, months, years, or decades) during which the rate of change of the injury can vary, (b) manifests as a prolonged or gradual decrease of renal tubular cell function or glomerular filtration rate (GFR) during which the rate of change of the function or rate can vary, and/or (c) manifests as a prolonged or gradual worsening of renal tubular cell injury during which the rate of change of the injury can vary. Chronic renal injury is distinct from any kidney condition, dysfunction or injury that is caused by a sudden or rapidly terminating event (e.g., occurring instantaneously, or over the course of seconds, minutes, hours, or days). In particular, chronic renal injury is distinct from any acute kidney condition, dysfunction or injury, (1) including but not limited to acute renal failure ("ARF"), and (2) such as, for example, addressed in and detected by the NGAL-based assays, methods and kits discussed in US 2004/0219603, US 2005/0272101, PCT WO 2005/121788, and PCT WO 2004/88276 (incorporated herein by reference in their entireties).

As used herein, a chronic renal injury includes or is caused by (by example but not by limitation) chronic infection, chronic inflammation, glomerulonephritides, vascular disease, interstitial nephritis, a drug (e.g., anticancer agent or other medicine), a toxin, trauma, a renal stone, long standing hypertension, diabetes, congestive heart failure, nephropathy from sickle cell anemia and other blood dyscrasias, nephropathy related to hepatitis, HIV, parvovirus and BK virus (a human polyomavirus), cystic kidney disease, congenital malformation, obstruction, malignancy, kidney disease of indeterminate cause, lupus nephritis, membranous glomerulonephritis, membranoproliferative glomerulonephritis, focal glomerular sclerosis, minimal change disease, cryoglobulinemia, Anti-Neutrophil Cytoplasmic Antibody (ANCA)-positive vasculitis, ANCA-negative vasculitis, amyloidosis, multiple myeloma, light chain deposition disease, complications of kidney transplant, chronic rejection of a kidney transplant, chronic allograft nephropathy, and the chronic effect of immunosuppressives.

The phrase "chronic renal injury status" as used herein means an assessment or diagnosis of the presence and/or extent of chronic renal injury in a mammal. This includes but is not limited to, for example, any clinical diagnosis of chronic renal injury or the absence thereof, any diagnosis based on K/DOQI guidelines, and any assessment using the present invention and based on the level of NGAL in the body sample to characterize the mammal as having "normal kidney function", "mild chronic renal injury", or "advanced chronic renal injury".

As used herein, "progressive renal disease", "worsening renal disease", "advanced chronic kidney injury", "advanced chronic kidney disease", "progressive renal injury", "worsening kidney injury", as well as other synonymous phrases, relate to a renal injury status wherein the injury may rapidly progress or worsen to renal failure, and typically indicates immediate hospitalization and/or treatment of the kidney injury to improve or ameliorate the kidney function.

As used herein, the expressions "early" or "sub-clinical" renal or kidney injury or damage means renal injury or damage that occurs prior to the rise in serum creatinine, or even prior to the development of urinary proteinuria.

As used herein, the term "about" refers to up to approximately a +/-10% variation from the stated value. The words "a" and "an" refer to "one or more".

The term "organ" means a differentiated biological structure comprised of cells and tissues that perform a certain function or functions in an organism.

A "mammal" or "mammalian subject" as used herein means a warm-blooded animal, e.g., from which a urine sample is obtained. Illustrative mammals include without limitation humans, non-human primates, pigs, cats, dogs, rodents, lapins, horses, sheep, cattle, goats and cows. The methods, assays, and kits according to the invention are particularly suited for humans.

"Improving" as used herein in the context of the methods of the invention refers to any measurable decrease in NGAL amount (e.g., NGAL level), or diminution or reversal of symptoms or other physiological evidence of chronic renal damage (e.g., based on GFR, serum creatinine levels, urine protein secretion levels, and the like).

"Worsening" as used herein in the context of the methods of the invention refers to any measurable increase in NGAL amount (e.g., NGAL level), or increase of symptoms or other physiological evidence of chronic renal damage (e.g., based on GFR, serum creatinine levels, urine protein secretion levels, and the like).

### 1. KIDNEY NGAL AND CIRCULATING NGAL AS BIOMARKERS

Kidney NGAL is produced by the nephron in response to tubular epithelial damage and is a marker of tubulointerstitial (TI) injury. It has been well established in acute renal failure (ARF) from ischemia or nephrotoxicity that NGAL levels rise in the urine of subjects, even after mild "subclinical" renal ischemia, in spite of normal serum creatinine levels. As described herein, kidney NGAL is expressed by the chronic renal disease kidney of various etiologies, and elevated kidney NGAL levels in urine are highly predictive of progressive kidney failure. NGAL was therefore assessed as further described herein in a longitudinal fashion as a non-invasive, early onset biomarker of kidney function decline in patients with chronic renal disease, and compared with proven biomarkers of kidney disease progression. In addition, a series of pathology studies also was conducted in order to evaluate the characteristics of kidney NGAL expression in the damaged kidney.

It had been previously demonstrated that expression of kidney NGAL is markedly increased by kidney tubules very early after ischemic or nephrotoxic injury in both animal and human models. Kidney NGAL is rapidly secreted into the urine, where it can be easily detected and measured, and precedes the appearance of any other known urinary or serum markers of ischemic injury. NGAL is resistant to proteases, suggesting that it can be recovered in the urine as a faithful biomarker of tubule expression of NGAL. Further, any NGAL derived from outside of the kidney, for example, filtered from the blood (denoted hereinafter as an "extra-renal pool" of NGAL or as "circulating" NGAL) does not appear in the urine of a healthy kidney, but rather is quantitatively taken up by the proximal tubule. Because of these characteristics we have previously proposed kidney NGAL as a urinary biomarker predictive of acute renal failure (see, e.g., US Patent Application 2004/0219603 and PCT International Application WO 2004/88276). We previously had shown that kidney NGAL is 100% specific and 99% sensitive for the development of ARF after cardiac surgery in pediatric patients. Similar data has also been obtained in a study of adult patients undergoing cardiac revision.

It has also been previously demonstrated that NGAL is expressed into the circulating blood system after an ischemic or nephrotoxic injury in both animal and human models. This "circulating NGAL" is believed to be an indirect response to injury to the renal tubular cells, and is believed to be expressed in the liver or other organ in response to renal tubular cell injury. Since it has been shown in animal models of renal tubular cell injury that the renal vein contains no or negligible levels of NGAL, it appears that the urine and serum carry distinct pools of NGAL, either of which can be predictive of renal tubular cell injury, and in particular of ischemic and nephrotoxic injury, as well as chronic injury.

While either kidney NGAL or circulating NGAL can be predictive of acute renal failure, it has now been found and demonstrated as described herein to also be predictive of early or sub-clinical renal injury, and of worsening kidney function in the chronic renal disease population. Given the expected doubling of chronic renal disease incidence and prevalence around the globe, and the cost that end-stage renal disease care represents, it is advantageous to identify either or both kidney and circulating NGAL as a biomarker that can be used to predict which patients are at an elevated risk of renal disease progression, so that early therapeutic interventions can be started, and so that medical regimens can be analyzed in a timely fashion. The present invention provides among other things a better understanding of the biological and clinical implications of kidney and circulating NGAL on chronic renal disease patients.

A primary benefit that identification of subclinical kidney damage can confer is the ability to initiate early intervention (e.g., medical treatments and/or procedures) to promote kidney function preservation and/or restoration. It has previously been shown that the presence and level of NGAL in either urine or serum, occurs and rises before serum creatinine in acute renal failure models both in mice and in humans, and can be elevated even when tubular damage is not evident by changes in serum creatinine, such as after sub-therapeutic doses of cisplatin.

NGAL is a small secreted polypeptide that is protease resistant and consequently readily detected in the urine and serum as a result of chronic renal tubule cell injury, typically in direct proportion to the degree and severity of the injury. Incremental increases in kidney or circulating NGAL levels in chronic renal failure patients over a prolonged period of time are diagnostic of worsening kidney disease. This increase in NGAL precedes and correlates with other urinary and circulating indicators of worsening chronic renal failure, such as increased serum creatinine, increased urine protein secretion, and lower glomerular filtration rate (GFR). Proper detection of worsening (or improving, if treatment has been instituted) renal status over time, confirmed by pre- and post-treatment NGAL levels in the patient, can aid the clinical practitioner in designing and/or maintaining a proper treatment regimen to slow or stop the progression of chronic renal failure. For example, in acute tubular necrosis (ATN), where kidney NGAL has been primarily studied, its rise anticipates that of serum creatinine by 24-48 hours. In the present invention, it has been determined that kidney NGAL also rises before the serum creatinine in chronic renal disease as well. Further, kidney NGAL is expressed in response to renal tubular cell injury and is excreted into the urine. Concurrently, circulating NGAL is expressed extra-renally into the bloodstream. Typically, NGAL is excreted at a higher concentration in urine than in blood for a particular event.

Urinary NGAL sampling is advantageous as non-invasive. Kidney NGAL concentration in urine is positively correlated with serum creatinine, indicating an association between NGAL levels and the extent of tubular damage. In the present invention, it is determined through rigorous clinical and pathological studies that the presence of kidney NGAL can both signal early kidney damage and aid in the detection of progression of chronic renal damage caused by progressive disease.

Circulating NGAL sampling is advantageous as blood sampling is and has been a routine clinical procedure, and blood samples of individuals have been and continue to be readily stored and preserved, providing a valuable database of historical samples that may be used to predict the progression of chronic renal injury in certain patients.

NGAL levels can be measured in patients undergoing therapeutic regimens which control blood pressure, blood glucose, renal hypertension and diets which limit protein intake, all therapies that are known to reduce the rate of progression of chronic renal disease. NGAL levels can be measured during the course of treatment for active glomerulonephritis or glomerulopathy which are chronic diseases of both the renal tubular and renal interstitial compartments. NGAL levels should typically decline during therapy for lupus nephritis, membranoproliferative glomerulonephritis, membranous glomerulonephritis, focal glomerulosclerosis, minimal change disease, cryoglobulinemia, and nephropathy related to hepatitis, HIV, parvovirus and BK virus. NGAL levels are measured and typically decline during treatment for lead cadmium, urate, chemotherapy related nephrotoxicity. Further, NGAL levels are measured and typically decline during treatment for polycystic and medullary cystic kidney disease, as well as for diabetes and hypertension.

### a. NGAL Expression in Normal Kidneys

We have extensively studied NGAL in humans, mice, and rats with normal renal function and in acute renal disease. We found that NGAL is normally secreted into the circulation by the liver and spleen, and it is filtered by the glomerulus and then recovered by the proximal tubule. Here, where NGAL is degraded in lysosomes (from 23KDa to 14KDa), and ligands located in the NGAL calyx are released. The capture of circulating, non-kidney NGAL by the proximal tubule is very effective, as little, if any NGAL is found in the urine of normal humans and mice [in humans: filtered load = (21ng/mL circulating NGAL) x (GFR), whereas urinary NGAL = 22 ng/mL. In the mouse: filtered load = (100ng/ml circulating NGAL) x (GFR), whereas urinary NGAL = 40 ng/ml]. Even after massive overload of the NGAL protein by systemic injections of NGAL (1mg), there is little protein recovered in the urine. The uptake into the proximal tubule likely reflects the action of megalin. This was ascertained in a megalin knockout mouse that contains a marked increase in the injected NGAL in the urine. Only a small amount of degraded NGAL (14 kDa) is found in the urine, reflecting processing within the kidney. We calculated a plasma t_{1/2} ~10 min that is likely the result of renal clearance. These data stress the specificity of urinary NGAL (NGAL recovered from urine) as a biomarker of kidney-expressed NGAL.

### b. NGAL Expression in Models of Acute Renal Failure

In acute diseases such as sepsis and surgical manipulations, including ischemia of the kidney, circulating NGAL levels rose 10³-10⁴ fold. We previously found that biopsies of human kidney with acute renal failure showed extensive NGAL immunopositive vesicles. These are presumably endocytic vesicles, and they co-localize with markers of lysosomes. Hence in the normal, as in acute renal failure, it appears that an extra-renal pool of NGAL delivers the protein to the proximal tubule where it is captured.

Remarkably, circulating NGAL protects renal function even after a severe model of ischemia. Filtered NGAL induces heme-oxygenase in the proximal tubule, a critical enzyme that maintains the viability of the tubule in the face of different types of stresses, suggesting a mechanism of protection.

In addition to the "extra renal pool" of NGAL (reflected in proximal tubule capture of NGAL), kidney epithelia also express the NGAL protein. In a normal healthy kidney, there is trace expression in distal tubules. However within 2-6 hours of cross clamping the renal artery or the ureter of mice, rats, pigs, or the kidneys of patients suffering acute renal failure, the renal tubule itself expresses NGAL. By real-time PCR, we found that NGAL mRNA rises 10³ fold. By *in situ* hybridization in mouse kidney, we found that ischemia induces massive expression of NGAL RNA in the ascending thick limb of the loop of Henle.

Likewise, urinary obstruction induces massive expression of NGAL mRNA in the collecting ducts. In the urine of mice, pigs and humans we detected a 10³-10⁴ fold increase in NGAL protein. A calculation of the fractional excretion of NGAL in human ATN was often greater than one (FE_{NGAL}>1), confirming that urinary NGAL reflected local synthesis rather than filtration from the blood. This was also the case in patients with prolonged renal failure who were initiating renal replacement therapy. The amount of urinary NGAL was so prodigious in these patients and its response to changes in renal function so rapid that we have used urinary NGAL as a sensitive and predictive marker of acute renal failure in children and in adults undergoing cardiac procedures.

Data shows that in addition to the "extra-renal pool" of NGAL that is cleared by the proximal tubule, renal epithelia expresses massive quantities (the "intra-renal pool") of NGAL that are secreted into the urine. Urinary NGAL is a specific and sensitive marker of acute epithelial damage and indeed it is a reversible marker. Treatment of ischemic mouse kidney with NGAL not only practically negated the rise in creatinine but it also reduced expression of intra renal (kidney) NGAL message by 70%.

### c. NGAL Expression in a Model of Chronic Renal Disease

It is notable that urine from patients with chronic renal failure contained much more NGAL than was present in the serum (even when corrected for urine creatinine level). This suggests that NGAL not only reflects acute changes in the tubulointerstitial compartment, but also chronic disease. In addition, it was found that NGAL is one of the most expressed proteins in the 4/5 nephrectomy model of chronic renal disease in two different animal lines. These preliminary data indicate that on the pathological level NGAL is a potent marker of CRD.

### d. Kidney NGAL distinguishable from Circulating NGAL

An analysis was made of the isoelectric point (pI) of kidney NGAL isolated from the urine of patients having ARF and CRD, and compared with the isoelectric point of NGAL isolated from neutrophils (i.e., circulating NGAL). Circulating NGAL has a pI of 8.5-9.2, while kidney NGAL from both ARF and CRD had a more complex pI of 6.9, 8.2, and 8.8-9.2. This suggests that the kidney NGAL and the circulating NGAL are distinctly glycosylated, and hence derived from different sources. This supports the assumption that kidney NGAL is generated by the renal tubule in response to injury, while circulating NGAL is generated by another organ in response to the same injury.

Distinguishing kidney NGAL from circulating NGAL in a body fluid can be useful in diagnosing any kidney injury, and the extent thereof. NGAL found in the urine is predominantly kidney NGAL, but can include some proportion and level of circulating NGAL, which is normally filtered and reabsorbed completely in a healthy kidney, but which may leak through into the urine in an injured kidney. Consequently, any urinary NGAL is typically predictive of kidney injury.

### 2. NGAL METHODS AND ASSAYS ACCORDING TO THE INVENTION

The assay of NGAL according to the invention can be performed on a body fluid sample from any mammal. For the purposes of the present invention, a mammalian subject experiencing acute renal injury will typically have present in both the urine and the blood stream a significant amount or level of NGAL protein, which can overwhelm the presence of any NGAL present in the body fluid as a consequence of a sub-clinical renal injury or a stable chronic renal injury. Consequently, the practice of the present invention typically involves the selection or identification of a mammalian subject that is not experiencing an acute renal injury. Typically, the clinician or physician can determine clinically whether or not a subject is experiencing an acute renal injury, by means well known in the art, such as by excluding recent events such as surgeries, ischemia, dehydration, sepsis, and nephrotoxin use.

The measured NGAL can originate not only from damaged kidney tubule cells, but also from activated circulating neutrophils. For example, it has been shown that serum NGAL levels are increased in inflammatory clinical settings such as severe bacterial or viral infections, acute severe peritonitis, and acute pulmonary exacerbations of cystic fibrosis. Given the possibility of neutrophilic NGAL expression, particularly in the blood stream, the subject is also typically evaluated clinically to determine if the subject is experiencing another condition that may contribute significantly to the level of NGAL in the sample. Such condition can include, but is not limited to, an acute bacterial or viral infection, acute inflammation including inflamed epithelia, an acute or chronic injury to another organ, and a cancer. In general, each of these conditions can be identified in a subject by standard clinical assessment, and are not typically associated with kidney injury.

There may be alternative approaches to evaluating a sample of serum or plasma that has been drawn from a subject that has some level of NGAL contributed from activated circulating neutrophils or from some other condition unrelated to kidney injury. One approach is to attempt to subtract a predicted amount of NGAL contributed by such source from the total NGAL level. Another approach is to set a minimum level or other predetermined level, in the hope of excluding samples such conditions that do not effect or cause kidney injury.

Further, a mammalian subject experiencing an acute bacterial or viral infection or an acute body inflammation will typically have present in the blood stream an increased amount or level of NGAL protein, which can disguise or overwhelm the presence of any NGAL present in the serum or plasma as a consequence of a sub-clinical, stable, or advanced chronic renal injury. Consequently, the practice of the present invention typically involves the selection of identification of a mammalian subject that is not experiencing an acute bacterial or viral infection or acute inflammation that can elevate the level or circulating NGAL in the blood. Alternatively, with the knowledge that a subject is experiencing an acute bacterial or viral infection of some degree, the clinician or physician can factor that contribution of circulating NGAL into the total assayed level of NGAL in assessing the renal injury status. Typically, the clinician or physician can determine clinically whether or not a subject is experiencing an acute bacterial or viral infection or inflammation by means well known in the art (e.g., white blood cell count, bacterial culture, and the like).

Further, a subject experiencing an acute or chronic injury to another organ, other than the kidney, will typically have expressed into, and have present in, the blood stream an increased amount or level of NGAL protein, which can disguise or overwhelm the presence of any NGAL present in the serum or plasma as a consequence of a sub-clinical, stable, or advanced chronic renal injury. Consequently, the practice of the present invention typically involves the selection or identification of a subject that is not experiencing an acute or chronic injury to another organ, other than the kidney, which can elevate the level of circulating NGAL in the blood. Alternatively, with the knowledge that a subject is experiencing an acute or chronic injury to another organ, of some degree, the clinician or physician can factor that contribution of circulating NGAL into total assayed level of NGAL in assessing the renal injury status. Typically, the clinician or physician can determine clinically whether or not a subject is experiencing an acute or chronic injury to another organ, other than the kidney, by means well known in the art.

Further, while it has been shown that a healthy kidney can clear effectively and quantitatively circulating NGAL from the blood stream, it is not known how this role is affected by a chronically injured kidney, and any resulting accumulation (gradual or rapid) of circulating serum NGAL.

### a. Sampling of body fluid

Methods well known in the art for collecting, handling and processing urine, blood, serum and plasma, and other body fluids, can be used in the practice of the present invention. Typically, though not by necessity, two or more consecutive or subsequent samples of a body fluid can be taken by similar means, such as the time of day, the quantity of sample drawn or collected, and the means for handling and processing the sample.

Depending upon the circumstances, including the level of NGAL in a sample and the clinical condition of the patient, the subject's body fluid can be sampled daily, or weekly or within a few weeks, or monthly or within a few months, semi-annually, or annually, and at any interval in between. Repeat sampling can be done at a period of time after treatment to detect any change in chronic renal injury status and to identify the extent of chronic renal injury over time. Sampling need not be continuous, but can be intermittent (e.g., sporadic). The period of time between intermittent sampling intervals is dictated by the condition of the subject, and can range from a sample taken continuously to a sample taken every ten years.

*b. Renal tubular cell injury, or renal injury, status*

The health status of a subject's kidney can be diagnosed by evaluating or comparing the level of NGAL assayed in a body fluid sample. In one embodiment, the renal tubular cell injury status of the subject is evaluated based on the mere presence of NGAL in the body fluid, as determined by an assay or other detection means. In another embodiment, the renal tubular cell injury status of the subject is evaluated based on the level of NGAL in the body fluid, as detected or determined by an assay or other detection means.

In another embodiment, the renal tubular cell injury status of the subject is evaluated based not only on NGAL levels, but also on the absence of an acute renal injury, or an acute bacterial or viral infection, acute inflammation, or acute or chronic injury to another organ, as determined by clinical evaluation. Such conditions are clinically evaluated at the time of the initial and any subsequent samples. Likewise, other co-morbidities, medications and primary or secondary events that occur between NGAL samples are evaluated and the effects factored into the results of the sampling.

The levels of NGAL determined in urine samples and serum samples were found to generally correspond with the assayed level of other well known and accepted biomarkers for chronic renal disease or injury, found in the subject sample, including serum creatinine, cystatine C, and eGFR. The level of NGAL determined can be expressed as the renal injury status of the patient, along with such other factors as the NGAL level from the subjects' prior sample, the time period between successive samples, or between an event and the sampling time, and any clinical assessment of acute renal injury, acute bacterial or viral infection, acute inflammation, and other organ injury.

The specific levels of NGAL described below in a serum and urine sample are determined using the NGAL ELISA methods described in Example 1a (SERUM) and 1b (URINE), respectively. Determinations of NGAL levels in serum and urine samples using such ELISA methods should yield similar results. Analysis of a sample by western blot provided a level of NGAL comparable to our NGAL ELISA. It should be understood that a determinations of NGAL levels in serum and urine samples using a different assay or assay methodology may result in a different absolute level of NGAL in the sample. Consequently, the invention includes levels of NGAL, for the purpose of evaluating renal injury, determined by a different assay which are equivalent to the levels of NGAL described herein using the NGAL ELISA methods described in Example 1a (SERUM) and 1b (URINE).

As described herein, a level of up to a base cut-off level of NGAL, typically from 0 to about 40 ng/mL, and more typically about 20 ng/mL, in a urine sample from a mammalian subject not experiencing another disease, disorder or condition that would elevate NGAL urine levels (e.g., acute kidney injury or kidney infection) indicates healthy kidney function of that subject. Furthermore, NGAL levels at or above an intermediate cut-off level, typically, between about 35 ng/mL to about 60 ng/mL urine, and more typically about 45 ng/mL, and up to an upper cut-off level, typically from about 120 ng/mL to about 150 ng/mL, indicate sub-clinical or stable chronic renal injury status. Further, NGAL levels at or greater than the upper cutoff level (e.g., greater than: about 120 ng/mL, about 135 ng/mL, about 140 ng/mL, about 155 ng/mL, about 160 ng/mL, about 170 ng/mL, about 180 ng/mL, about 190 ng/mL, or about 200 ng/mL) tend to indicate advanced or worsening chronic renal injury status, and/or a greater risk of progressing to chronic renal failure.

As also described herein, a level of up to a base cut-off level of NGAL, typically from 0 to about 40 ng/mL, and more typically about 20 ng/mL, in a serum (or plasma) sample from a mammalian subject not experiencing another disease, disorder or condition that would elevate NGAL serum levels (e.g., acute kidney injury, acute bacterial or viral infection, acute inflammation, an acute or chronic injury of some other organ, or cancer) indicates healthy kidney function of that subject. Furthermore, NGAL levels at or above an intermediate cut-off level, typically, between about 35 ng/mL to about 60 ng/mL serum (or equivalent level in plasma), and more typically about 45 ng/mL, and up to an upper cut-off level, typically from about 150 ng/mL to about 250 ng/mL, indicate sub-clinical or stable chronic renal injury status. Further, NGAL levels at or greater than the upper cutoff level (e.g., greater than: about 150 ng/mL, about 160 ng/mL, about 170 ng/mL, about 180 ng/mL, about 190 ng/mL, about 200 ng/mL, about 210 ng/mL, about 220 ng/mL, or about 230 ng/mL, or greater) tend to indicate advanced or worsening chronic renal injury status, and/or a greater risk of progressing to chronic renal failure.

In a further method of assaying the renal status, the assayed level of NGAL in a urine, serum or plasma sample from a subject having healthy kidney function or a degree of renal injury, as described earlier, can be correlated with the GFR to assess the stage of chronic kidney disease.. The level of glomerular filtration rate (GFR) is widely accepted as the best overall measure of kidney function in health and disease. Providers and patients are familiar with the concept that "the kidney is like a filter". GFR is the best measure of the kidneys' ability to filter blood, and thus, function. Consequently, a correlation between the level of NGAL in urine, serum and plasma, and GFR, would establish NGAL as an excellent biomarker that can predict the subjects GFR result, and thus assist in the prediction and diagnosis of the subjects' renal injury status, and help guide intervention and treatment options.

**Table 1 shows a correlation between GFR and the stage of CRD. The level of NGAL in serum has been shown to correlate very well with GFR, particularly in a patient with advanced CRD (that is, one having a higher CRD stage or lower (<30) GFR value).**

| | TABLE 1 | |
|---|---|---|
| STAGE | DESCRIPTION | GFR (mL/min/1.73 m²) |
| (null) | At increased risk | ≥90 (with CRD risk factors) |
| 1 | Kidney damage with normal to high GFR | > 90 |
| 2 | Kidney damage with mildly reduced GFR | 60-89 |
| 3 | Moderate reduced GFR | 30-59 |
| 4 | Severe reduced GFR | 15-29 |
| 5 | Kidney failure | < 15 (or dialysis) |

NGAL also has been shown, as provided herein, to correlate with the level of cystatin C. As an exact measure of the GFR is the primary prerequisite for identification of the renal injury status, and for the staging and treatment of CRD, NGAL emerges as an outstanding biomarker for the assessment of kidney injury and its progress, and enables improved and more timely therapies and interventions.

With an expanding population of human subjects having early stage CRD, there remains a need to better track and record the level of early CRD biomarkers throughout the lifecycle of the human population. The present invention provides a means for obtaining a historical profile of NGAL levels in serum, plasma and urine, which can then help the patient and the physician to identify events and lifestyles factors that can adversely affect, or ameliorate, renal health. Individuals who may not have any chronic renal injury but who are at an increased risk, e.g., due to lifestyle factors or injury-causing events, can be assessed as part of a routine health encounter, based on the levels of NGAL in their body fluids.

As a subject deteriorates in kidney health into sub-clinical CRD, more frequent evaluations should be made, based on more frequently assayed samples and the NGAL levels assayed therefrom. The more frequent evaluations in turn can precipitate an evaluation of the root cause of the chronic kidney injury, and an earlier therapeutic intervention designed to improve renal health or slow the deterioration of renal health caused by of chronic renal injury.

The present invention is as also particularly useful in the evaluation and assessment of a therapeutic program for the treatment of a CRD. The attending physician can prescribe periodic assays that are sampled at or after a therapeutic treatment, and more typically periodically after a therapeutic treatment, in order to evaluate a change in the renal status as a result of the treatment.

### 3. OTHER KIDNEYNGAL ASSAY CONSIDERATIONS

The present invention employs detection of an NGAL biomarker in methods, assays, and kits, as well as components employed in same.

In general, detection of NGAL according to the invention relies on forming a complex of NGAL and an antibody against NGAL (so-called capture antibody), and then optionally detecting the NGAL by contacting the complex with a second antibody for detecting the biomarker or the capture antibody. The detectable antibody can be labeled with a detectable marker or means for detection, such as a radioactive label, enzyme, biological dye, magnetic bead, or biotin, as is well known in the art.

Typically according to the invention the step of detecting (e.g., determining) the presence or quantity (level or concentration) of NGAL in the body fluid sample comprises: contacting the body fluid sample with an antibody for NGAL to allow formation of an antibody-NGAL complex, and determining the presence and/or quantity of the antibody-NGAL complex. The quantity of antibody-NGAL complex is a function of the quantity of NGAL in each sample. The step of contacting the fluid sample with an antibody for NGAL to allow formation of an antibody-NGAL complex typically involves the step of contacting the sample with a media (e.g., solid support or solid phase) having affixed thereto the antibody.

Typically the step of determining the presence or quantity of the antibody-NGAL complex in the body fluid sample involves contacting the complex with a second antibody for detecting NGAL. Taken further, this step optionally can include the steps of: separating any unbound material of the sample from the antibody-NGAL complex, contacting the antibody-NGAL complex with a second antibody for NGAL to allow formation of a NGAL-second antibody complex, separating any unbound second antibody from the NGAL-second antibody complex, and determining the quantity of the NGAL-second antibody complex in the sample, wherein the quantity of the NGAL-second antibody complex in the sample is a function of the quantity of the antibody-NGAL complex in the sample.

Still further, the step of determining the quantity of the NGAL-second antibody complex in the sample can include methods well-known in the art, including the steps of: adding Horseradish peroxidase (HRP)-conjugated streptavidin to the sample to form a complex with the NGAL-second antibody complex, adding a color-forming peroxide substrate to the sample to react with the HRP-conjugated streptavidin to generate a colored product, and thereafter reading the color intensity of the colored product in an enzyme linked immunosorbent assay (ELISA) reader, wherein the color intensity is a function of the quantity of the NGAL-second antibody complex in the sample.

In addition to an NGAL ELISA assay as described in the Examples, other analytical methods can be used that provide satisfactory specificity, sensitivity, and precision, and can include a lateral flow device, and a dipstick. For example, a dipstick surface is coated with a capture antibody for NGAL, and an enzyme-labeled detection antibody against is used to detect NGAL that binds with the capture antibody. In general, any binding assay using the principles described herein and known in the art could be devised and used in accordance with the present invention to detect and monitor NGAL. In particular, a method and kit of the present invention for detecting the NGAL biomarker can be made by adapting the methods and kits known in the art for the rapid detection of other proteins and ligands in a biological sample. Examples of methods and kits that can be adapted to the present invention include those described in U.S. Pat. No. 5,656,503, issued to May et al. on Aug. 12, 1997, U.S. Pat. No. 6,500,627, issued to O'Conner et al. on Dec. 31, 2002, U.S. Pat. No. 4,870,007, issued to Smith-Lewis on Sep. 26, 1989, U.S. Pat. No. 5,273,743, issued to Ahlem et al. on Dec. 28, 1993, and U.S. Pat. No. 4,632,901, issued to Valkers et al. on Dec. 30, 1986, all such references being hereby incorporated by reference in their entireties for their teachings regarding same.

Both monoclonal and polyclonal antibodies that bind NGAL are useful in the assays, methods and kits of the present invention. The antibodies are available commercially or can be prepared by methods known in the art. Monoclonal antibodies for NGAL, are described, for example, in "Characterization of two ELISAs for NGAL, a newly described lipocalin in human neutrophils", Lars Kjeldsen et al., (1996) Journal of Immunological Methods, Vol. 198, 155-16, herein incorporated by reference in its entirety. Examples of commercially available monoclonal antibodies for NGAL include those obtained from the Antibody Shop, Copenhagen, Denmark, as HYB-211-01, HYB-211-02, and NYB-211-05. Typically, HYB-211-01 and HYB-211-02 can be used with NGAL in both its reduced and unreduced forms. An example of a polyclonal antibody for NGAL is described in "An Iron Delivery Pathway Mediated by a Lipocalin", Jun Yang et al., Molecular Cell, (2002), Vol. 10, 1045-1056, herein incorporated by reference in its entirety. To prepare this polyclonal antibody, rabbits were immunized with recombinant gel-filtered NGAL protein. Sera were incubated with GST-Sepharose 4B beads to remove contaminants, yielding the polyclonal antibodies in serum, as described by the applicants in Jun Yang et al., Molecular Cell (2002).

Likewise, purified NGAL in a variety of forms (e.g., recombinant human NGAL) for use as a standard and a calibrator material can be prepared such as is known in the art (e.g., as described in Kjeldsen et al. (1996)) or is commercially available.

The media (e.g., solid support or solid phase) used in the methods and assays of the invention can be any suitable support used in immunochemical analyses, e.g., including but not limited to polystyrene, polyvinyl chloride, or polyethylene surface or particles. Optionally, the media (e.g., solid support or solid phase) includes one or more electrodes to provide for detection based on electrochemical interactions (e.g., US Patent No. 6,887,714).

A kit for use in the method of the invention typically comprises a media (e.g., solid support or solid phase) having affixed thereto the capture antibody, whereby the body fluid sample (e.g., urine, serum or plasma sample) is contacted with the media to expose the capture antibody to NGAL contained in the sample. The kit includes an acquiring means that can comprise an implement, such as a spatula or a simple stick, having a surface comprising the media. The acquiring means can also comprise a container for accepting the body fluid sample, where the container has a fluid sample-contacting surface that comprises the media. In another typical embodiment, the assay for detecting the complex of the NGAL and the antibody can comprise an ELISA, and can be used to quantitate the amount of NGAL in a body fluid sample. In an alternative embodiment, the acquiring means can comprise an implement comprising a cassette containing the media. In all cases, however, a kit typically includes instructions for its use, as well as any additional information (e.g., storage, safety or other information) regarding the kit components.

Alternately, the methods, kits, and assays of the present invention can be adapted for use in automated and semi-automated systems (including those wherein the solid phase comprises a microparticle), as described, e.g., in US Patent Nos. 5,089,424 and 5,006,309, and as, e.g., commercially marketed by Abbott Laboratories (Abbott Park, IL) including but not limited to Abbott's ARCHITECT®, AxSYM, IMX, PRISM, Quantum II, as well as other platforms.

Thus, in addition to others, the present invention provides a kit for use in the early detection of chronic renal injury in a mammal, based on assessing the body fluid sample (e.g. urine or serum) of a subject, comprising one or more of the following: 1) a means for acquiring a quantity of a body fluid sample (e.g., sample collection container or vial); 2) a media having affixed thereto a capture antibody capable of complexing with NGAL (e.g. dipstick or microtiter plate); 3) assay components for the detection of a complex of NGAL and the capture antibody (e.g., detection antibody, wash solution, incubation solutions, detection solutions, calibrators, controls, and the like); 3) kit instructions; and 4) other literature describing the kit components. Further, according to the invention, the body fluid sample acquiring means optionally (a) comprises the media on its body fluid-contacting surface, and/or (b) comprises an implement comprising a cassette containing the media.

In one embodiment of the invention, the kit optionally is a point-of-care kit. In such a point-of-care kit according to the invention the acquiring means optionally comprises an implement comprising a dip-stick, wherein the dip-stick surface comprises the media. Additionally, in a point-of-care kit the assay optionally comprises a colorimetric dip-stick assay.

Moreover, the invention provides a competitive enzyme linked immunosorbent assay (ELISA) kit for determining the chronic renal injury status of a mammalian subject, optionally comprising a first antibody specific to NGAL to detect its presence in a body fluid sample of the subject. Such a kit optimally can be employed wherein the body fluid sample (e.g., urine, serum, or plasma sample) comprises a fluid amount of about 1 milliliter or less.

The invention will be better understood through examples illustrating its use and efficacy. By way of example and not limitation, Examples of the present invention shall now be given.

### EXAMPLES:

### Example 1: ASSAYS AND METHODS

### a. NGAL ELISA - SERUM

Unless otherwise specified, the level of NGAL in serum is assayed with an ELISA as follows. Microtiter plates are coated overnight at 4°C with a mouse monoclonal antibody raised against human NGAL (#HYB211-05, Antibody Shop, Gentofte, Denmark). All subsequent steps were performed at room temperature. Plates are blocked with buffer containing 1% BSA, coated with 100 µl of serum or standards (NGAL concentrations ranging from 1-1000 ng/ml), and incubated with a biotinylated monoclonal antibody against human NGAL (#HYB211-01B, Antibody Shop) followed by avidin-conjugated HRP (Dako, Carpenteria, California, USA). TMB substrate (BD Biosciences, San Jose, CA) is added for color development, which is read after 30 min at 450 nm with a microplate reader (Benchmark Plus, BioRad, Hercules, California, USA). The inter- and intra-assay coefficient variations are 5-10%. All measurements are made in triplicate, and in a blinded fashion. Serum NGAL is measured as ng/ml, and can be expressed as log transformed values.

### b. NGAL ELISA - URINE

Unless otherwise specified, the level of NGAL in urine is assayed with an ELISA as follows. Microtiter plates are coated overnight at 4°C with a mouse monoclonal antibody raised against human NGAL (#HYB211-05, Antibody Shop, Gentofte, Denmark). All subsequent steps were performed at room temperature. Plates are blocked with buffer containing 1% BSA, coated with 100 µl of urine (centrifuged) or standards (NGAL concentrations ranging from 1-1000 ng/ml), and incubated with a biotinylated monoclonal antibody against human NGAL (#HYB211-01B, Antibody Shop) followed by avidin-conjugated HRP (Dako, Carpenteria, California, USA). TMB substrate (BD Biosciences, San Jose, CA) is added for color development, which is read after 30 min at 450 nm with a microplate reader (Benchmark Plus, BioRad, Hercules, California, USA). The inter- and intra-assay coefficient variations are 5-10%. All measurements are made in triplicate, and in a blinded fashion. Urinary (kidney) NGAL is measured as ng/ml, and can be expressed as log transformed values.

### c. STATISTICAL ANALYSIS OF RESULTS

A two-sample t-test or Mann-Whitney Rank Sum Test is used to compare continuous variables. Categorical variables are compared using the Chi-square test or Fisher's exact test. The associations between variables are assessed by Pearson correlation analysis. Comparison between correlations is done using Steiger's Z statistics by creating Z-scores from correlation coefficients. Residual analysis is performed to evaluate the agreement between different predictor variables (serum creatinine, eGFR, NGAL and cystatin C) and measured GFR. To measure the sensitivity and specificity for serum NGAL and cystatin C at various GFR cut-offs, receiver-operating characteristic (ROC) curves are generated using SAS MACRO program, and the SAS 9.1 statistical package is used in the analysis. The area under the curve (AUC) is calculated to ascertain the quality of NGAL and cystatin C as biomarkers. An AUC of 0.5 is no better than expected by chance, whereas a value of 1.0 signifies a perfect biomarker. Unless otherwise specified, values are presented as means ± SD. A **P** ≤ 0.05 is considered statistically significant.

### Example 2: (a) Urinary NGAL Expression in a Population of CKD Patients

Urinary NGAL levels were assessed in 91 outpatients from the general nephrology clinic at Columbia University Medical Center (CUMC) that were referred by outside nephrologists for treatment consultation. These were patients with kidney disease resulting from a spectrum of etiologies. Table 2 below shows their baseline characteristics. Mean age was 49.2 years and about half the cohort was female. The correlation coefficient between NGAL and other continuous parameters was determined by log transforming NGAL, along with the serum creatinine, urine albumin to creatinine ratio (UACR) and the total urinary protein. Log NGAL was found to correlate with log serum creatinine at the baseline visit (r = 0.54, p<0.0001), the change in serum creatinine between the baseline and follow-up visit (r = 0.49, p = 0.002), GFR (r = -0.22, p = 0.04), log UACR (r = 0.55, p < 0.0001), and the log of the total urinary protein (r = 0.61, p = <0.0001). There was no correlation between urinary NGAL and age (SD 17.0), systolic blood pressure (SD 15.8), diastolic blood pressure (SD 11.6), weight (SD 24.1), and serum albumin (SD 4.3).

| Table 2: Baseline Population Characteristics | |
|---|---|
| | |

| Demographics | Value |
|---|---|
| Age (years - Mean) | 49.2 |
| Female (%) | 47.8 |
| Race (%) | |
| White | 73.9 |
| Black | 10.2 |
| Hispanic | 4.6 |
| Asian | 8.0 |
| Other | 3.4 |
| | |
| Clinical Parameters | |
| Systolic Blood Pressure (mmHg - mean) | 135.4 |
| Diastolic Blood Pressure (mmHg - mean) | 81.6 |
| Weight (kg - mean) | 83.3 |
| | |
| Laboratory Parameters | |
| Urine NGAL (g/mL - mean) | 94.6 |
| Spot Urine Protein (mg/gm - mean) | 3.2 |
| Urine Albumin/Creatinine Ratio (mg/mg - mean) | 2,338.6 |
| Serum Creatinine (mg/dL - mean) | 2.6 |
| Serum Albumin (g/dL - mean) | 4.2 |
| Estimated GFR (mL/minute - mean) | 46.4 |

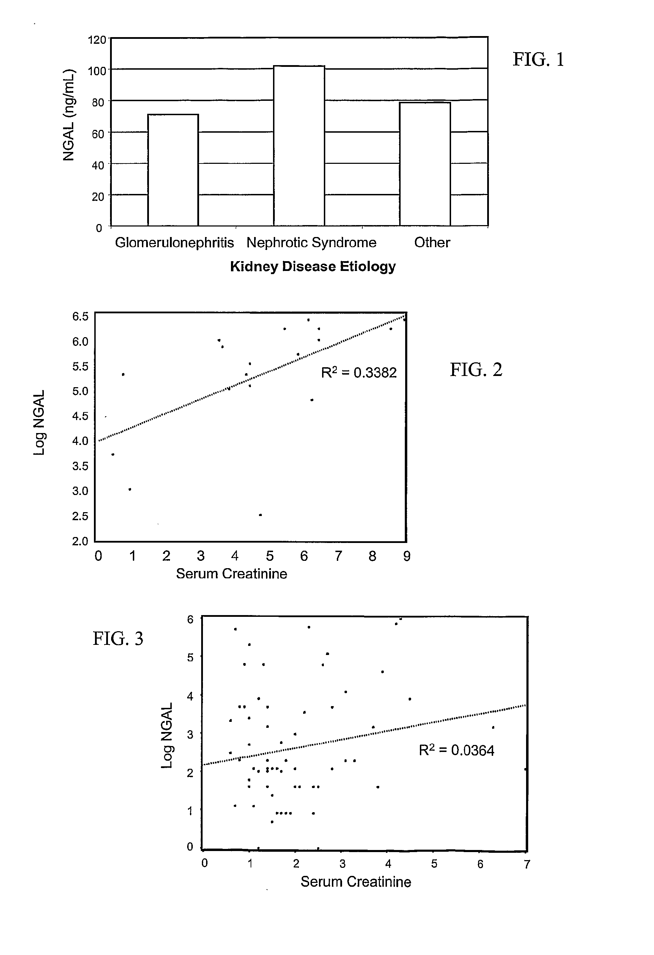

Table 3 lists the etiologies of CRD in this cohort. Out of 91 patients, only 81 had assigned diagnoses. The etiology of CRD consisted of 38% glomerulonephritis, 44% nephrotic syndrome, and 17% other causes. The mean urinary NGAL level for all patients was 94.6 ng/ml urine. Mean urinary NGAL levels by etiology of CRD were 71.2 ng/mL for the group with glomerulonephritis, 101.7 ng/mL for the group with nephrotic syndrome, and 78.2 ng/mL for the group with other etiologies of kidney disease (See FIG. 1). These levels were not statistically different from each other by ANOVA (F test = 0.6890).

| Table 3: Kidney Diagnoses by Pathological Subgroup | |
|---|---|
| | |

| Nephritic Syndrome (n=31) | Percent |
|---|---|
| Anti Cardiolipin Disease | 3.2 |
| C1q Nephropathy | 3.2 |
| Chronic glomerulonephritis (GN) | 6.5 |
| Fibrillary GN | 3.2 |
| Immunocomplex GN | 3.2 |
| IgA Nephropathy | 42.0 |
| Membranoproliferative GN | 6.5 |
| Rapidly Progressive Glomerulonephritis (RPGN) | 3.2 |
| Lupus Nephritis | 29 |
| | |
| Nephrotic Syndrome (n=36) | Percent |
| Amyloid | 2.8 |
| Focal Segmental Glomerulosclerosis (FSGS) | 47.2 |
| Minimal Change Disease | 16.7 |
| Membranous Nephropathy | 30.6 |
| Nephrotic Unspecified | 2.8 |
| | |
| Other (n=14) | Percent |
| CRD Unspecified | 28.5 |
| Diabetic Nephropathy | 28.6 |
| Lithium Toxicity | 14.3 |
| Polycystic Kidney Disease | 28.6 |

### b. Urinary NGAL Expression and its Relationship to Kidney Disease Progression Status

Table 4 demonstrates the baseline characteristics of the patients stratified on progression to the primary endpoint of a 25% or more increase in serum creatinine or the development of ESRD by the next follow-up visit. Follow-up information was obtained on 82 patients out of the original 91. 18 patients (22.0%) of the cohort reached the primary endpoint. Mean urinary (or "kidney") NGAL for patients reaching the endpoint was 294.6 ng/mL, while those who did not reach the endpoint had an NGAL level of 46.6 ng/mL (p < 0.0001). The group of patients who progressed to endpoint also had a significantly higher mean proteinuria, and a significantly lower mean GFR.

| Table 4: Population Characteristics by Progression Status | | | | | | | |
|---|---|---|---|---|---|---|---|
| | | Progressors | | | Non Progressors | | p-value |
| | n | | se | n | | se | |
| Demographics | | | | | | | |
| Age (years - Mean) | 16 | 54.4 | 3.57 | 64 | 49.4 | 2.15 | 0.3 |
| Female (%) | 10 | 55.6 | | 29 | 45.3 | | 0.6 |
| Race (%) | | | | | | | 0.2 |
| White | 12 | 70.6 | | 48 | 76.2 | | |
| Black | 1 | 5.9 | | 6 | 9.5 | | |
| Hispanic | 0 | 0 | | 4 | 6.4 | | |
| Asian | 4 | 23.5 | | 3 | 4.8 | | |
| Other | 0 | 0 | | 2 | 3.2 | | |
| Clinical Parameters | | | | | | | |
| Systolic Blood Pressure (mmHg - mean) | 16 | 141.3 | 4.45 | 63 | 133.7 | 1.97 | 0.1 |
| Diastolic Blood Pressure (mmHg - mean) | 16 | 83.3 | 2.35 | 63 | 81.0 | 1.56 | 0.3 |
| Weight (kg - mean) | 15 | 81.4 | 4.79 | 62 | 83.8 | 3.24 | 1.0 |
| Kidney Disease | | | | | | | |
| | | | | | | | |
| Diagnosis | | | | | | | 0.6 |
| Nephritic Syndrome (%) | 4 | 26.7 | | 25 | 42.4 | | |
| Nephrotic Syndrome (%) | 8 | 53.3 | | 23 | 39.0 | | |
| Other (%) | 3 | 20.0 | | 11 | 18.6 | | |
| | | | | | | | |
| Laboratory Parameters | | | | | | | |
| Urine NGAL (µ/dL - mean) | 18 | 294.6 | 46.02 | 64 | 46.6 | 10.90 | <0.0001 |
| Spot Urine Protein (mg/gm - mean) | 7 | 10.2 | 4.07 | 43 | 2.2 | 0.06 | 0.004 |
| Serum Creatinine (mg/dL - mean) | 18 | 4.8 | 0.56 | 63 | 2.0 | 0.16 | 0.0001 |
| Serum Albumin (g/dL - mean) | 13 | 3.4 | 0.26 | 58 | 4.4 | 0.65 | 0.2 |
| Estimated GFR (mL/minute - mean) | 15 | 29.0 | 10.05 | 62 | 49.3 | 3.86 | 0.001 |

Linear regression models were then constructed to assess the relationship between the urinary NGAL and renal function and proteinuria, stratifying on the outcome. In these models NGAL, serum creatinine, and the AUCR was log transformed to normalize the data's distributional properties. The regression coefficients are listed in Table 5. There was a significant linear relationship between log NGAL and log serum creatinine only for patients who progressed to the endpoint.

| Table 5: Regression Coefficients for Log NGAL and Kidney Parameters | | | | | | | | |
|---|---|---|---|---|---|---|---|---|
| | | Progressors | se | p-value | | Non-Progressors | se | p-value |
| Variable | | | | | | | | |
| Log Serum Creatinine | | 0.28 | 0.1 | 0.01 | | 0.23 | 0.1 | 0.1 |
| Total Proteinuria | | -0.07 | 0.02 | 0.03 | | 16.4 | 3.3 | < 0.0001 |
| Log UACR | | 0.32 | 0.23 | 0.2 | | 0.49 | 0.1 | < 0.0001 |

As seen in FIG. 2, in patients who progressed there is a significant linear association in the positive direction between NGAL and creatinine levels (R² = 0.3382). As seen in FIG. 3, the scatter of data points confirms the non-significant association of NGAL levels and serum creatinine in non-progressors (R² = 0.0364). Stated another way, NGAL levels are very good to have in progressors because they add prognostic information to the serum creatinine.

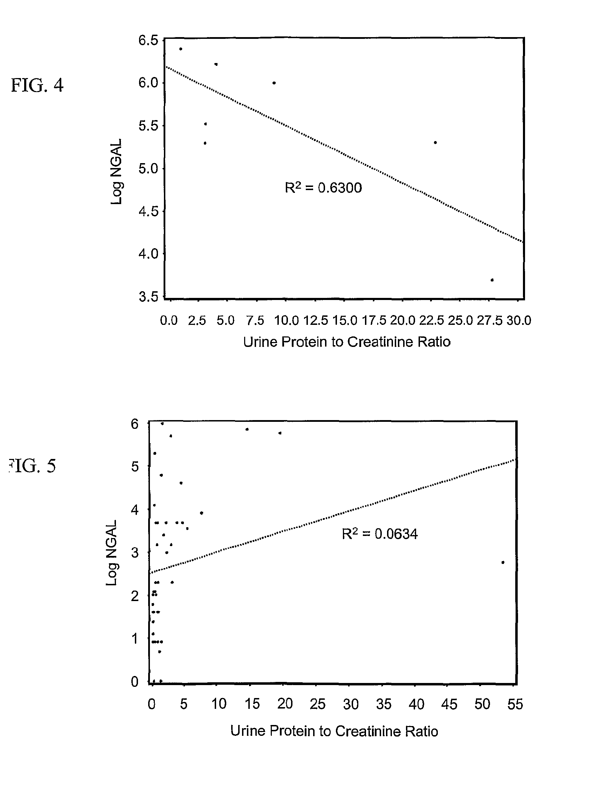

For total proteinuria, regression models demonstrated a significant inverse association between total proteinuria and log NGAL in patients reaching endpoint (FIG. 4 [R² = 0.6300] and FIG. 5 [R² = 0.0634]). There was a linear relationship between log NGAL and log UACR only in those patients that did not progress to endpoint.

### c. NGAL is Predictive of a Future Decline in Kidney Function

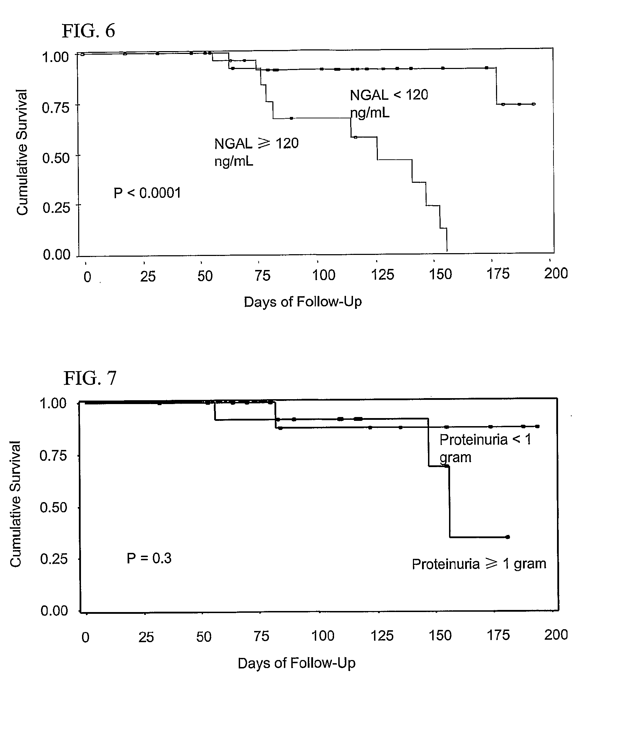

The elevation in urinary NGAL among patients that reached the endpoint led to the hypothesis that NGAL may be an independent predictor of renal function decline. A sensitivity analysis was conducted for both urinary NGAL and urinary protein, an important predictor of progressive renal failure. The primary endpoint was selected as a 25% increase in serum creatinine or the development of ESRD by the time of follow-up. The area under the curve (AUC) for NGAL was 0.908 and that for proteinuria was 0.833. The high end or upper cutoff value of NGAL level was then defined that gave the best sensitivity and specificity for NGAL total proteinuria. At an NGAL concentration 120 ng/mL, the sensitivity was 83.3% and the specificity was 85.9% for predicting the development of poorer renal function at the follow-up visit. For total urinary protein, a cutoff of 1 gram daily demonstrated a sensitivity of 85.7% and a specificity of 81.4%. Using this cutoff, Kaplan-Meier curves were constructed for both NGAL and proteinuria (FIGS. 6 and 7). As shown in FIG. 6, median survival time for the development of the primary endpoint was 125 days in group with a urinary NGAL ≥ 120 ng/mL (p < 0.0001). There was no difference in the survival curves for the group with and without proteinuria, as defined by a cutoff of 1 gm daily (FIG. 7, p = 0.3).

| Table 6: Hazard Models for the Association of NGAL Levels with Progressive Kidney Disease | | | | |
|---|---|---|---|---|
| Univariate Proportional Hazard Models | | Hazard Ratio | | p-value |
| NGAL (> 120 µg/dL) | | 12.4 | | 0.001 |
| | | | | |
| Serum Creatinine (mg/dL) | | 1.6 | | 0.002 |
| | | | | |
| GFR (mL/minute) | | 1.0 | | 0.2 |
| | | | | |
| Proteinuria (> 1 gram) | | 3.1 | | 0.3 |
| | | | | |
| Hypertension (SBP ≥ 140 or DBP ≥90) | | 2.7 | | 0.1 |
| | | | | |
| Multivariate Proportional Hazard Models | | Hazard Ratio | | p-value |
| NGAL (> 120 µg/dL) | | 8.4 | | 0.01 |
| Serum Creatinine (mg/dL) | | 1.2 | | 0.2 |

Further exploration by proportional hazard regression modeling revealed that at an upper cutoff value of 120 ng/ml urinary NGAL was the only independent predictor that remained significantly associated with worsening kidney function at follow-up in a multivariate model (HR 8.4, p < 0.01) (See Table 6).

### d. Alternative primary endpoint and cutoff value for NGAL

Using the same subjects, we then selected a primary endpoint of 50% increase in serum creatinine, or the development of end stage renal disease by the time of follow-up (122.1 days + 45.7 days). For serum creatinine, the area under the ROC was 0.783, and for proteinuria the area under the curve was 0.775. On this basis, we set an arbitrary upper cutoff value of 150 ng NGAL/mL of urine, that provided reasonable sensitivity (0.75), specificity (0.88), positive predictive value (0.63), and negative predictive value (0.93), to identify the maximum number of subjects who progressed to chronic renal failure. The sensitivity and specificity for NGAL measured in ng/mg of creatinine were 0.75 and 0.84, respectively.

### e. NGAL and its Relationship to Fibrosis on Kidney Biopsy

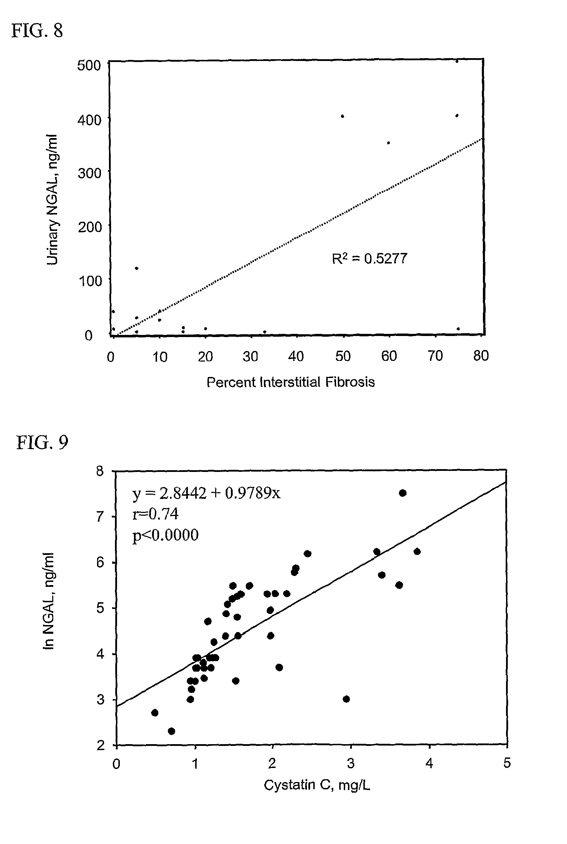

In order to evaluate the relationship between urinary NGAL levels and degree of fibrosis on kidney biopsy, we examined the results of fibrosis scores on 16 kidney biopsy specimens from the cohort of 91 patients. These 16 were chosen because they were read by the renal pathology department at CUMC. These biopsies were obtained up to 2 years prior to the urine NGAL level. Regression analysis indicated that urine NGAL levels obtained up to 2 years post-renal biopsy were highly correlated with the percent of fibrosis on biopsy (figure 8, r² = 0.53, p < 0.001). We believe this to suggest that NGAL levels are reflective of the chronicity of kidney damage. If this is true, then this is a pathological confirmation of its utility in predicting poor renal outcomes. Collectively, these data indicate an innovative, high-impact development in the discovery and characterization of NGAL as a predictive biomarker for the progression of chronic kidney disease.

### Example 3: RESULTS OF PATIENT STUDIES (SERUM)

### a. Circulating NGAL Expression in a Population of CRD Patients

Forty five consecutive children and adolescents (ages 6-21 years) with CRD stages 2-4 (measured GFR = 15-89 mL/min/1.73m²) were prospectively recruited between 2002 and 2004. The stages of CRD were defined according to the K/DOQI guidelines. None of the subjects received a kidney transplant during the study or were post-transplant. The medical records were reviewed for demographics, cause and duration of CRD, and medications.

Serum creatinine levels were measured using a kinetic, reflectance spectrophotometric assay (Vitros® 950 Chemistry System from Ortho Clinical Diagnostics, Raritan, New Jersey, USA) as part of routine care. Estimated GFR (eGFR) was calculated using the Schwartz formula. Kidney function at the time of enrollment in the study was also determined by measuring GFR using a single intravenous injection of Ioversol injection 74% (Optiray 350^{®}, Mallinckrodt Inc., St Louis, Missouri, USA). Iodine in timed blood samples was measured by X-ray fluorescence analysis (Renalyzer PRX90, Diatron AB Inc, Sweden) and GFR was calculated from the slope of the iodine disappearance curve. Serum cystatin C was measured at enrollment by a standardized and widely validated immunonephelometric method (Dade-Behring BN ProSpec System Version 1.1, Marburg, Germany). In comparing GFR measurements by sensitive nuclear tracer techniques and serum cystatin C in 62 patients with a variety of chronic kidney conditions, an excellent correlation has been documented between these techniques, and inter- and intra-assay coefficient variations of 5-10% (data not shown). All measurements were made in triplicate, and in a blinded fashion.

The sampling times were determined based on the eGFR. For subjects with eGFR > 60 mL/min/1.73 m², blood samples were obtained at 150, 195, and 240 minutes, for those with eGFR of 30-60 mL/min/1.73 m² at 150, 240, and 300 minutes, and for those with eGFR of < 30 mL/min/1.73 m² at 180, 270, and 360 minutes after Ioversol injection.

Serum NGAL was measured and statistically analyzed at enrollment using the NGAL ELISA described in the Methods and Assays section.

The main causes of CRD were renal dysplasia/obstructive uropathy (67%) and glomerular and cystic disease (33%). Almost half of the patients (46%) were taking antihypertensive medications. Of those on medications, all were taking angiotensin converting enzyme inhibitors (ACEI). Fourteen patients were taking an ACEI or an angiotensin receptor blocker as an anti-proteinuric agent. The mean duration of CRD was 8.8 ± 5.6 years. None of the subjects had CRD for less than 1 year. Thirteen (28%) patients had CRD stage 2, 19 (42%) stage 3, and 13 (28%) stage 4.

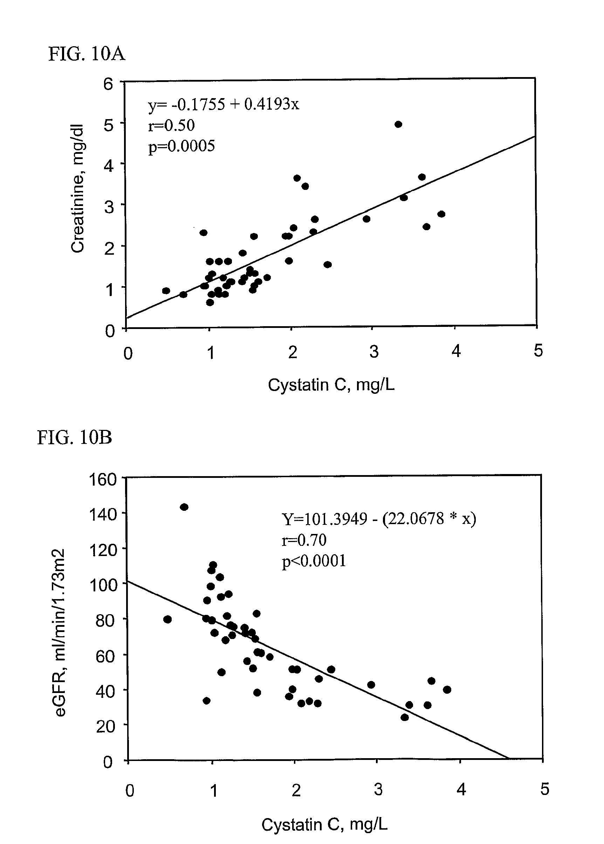

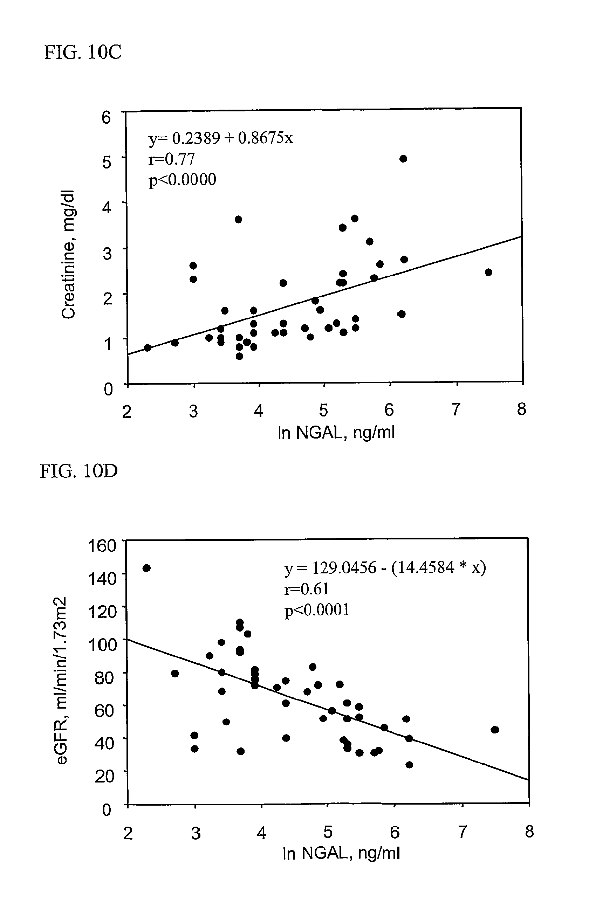

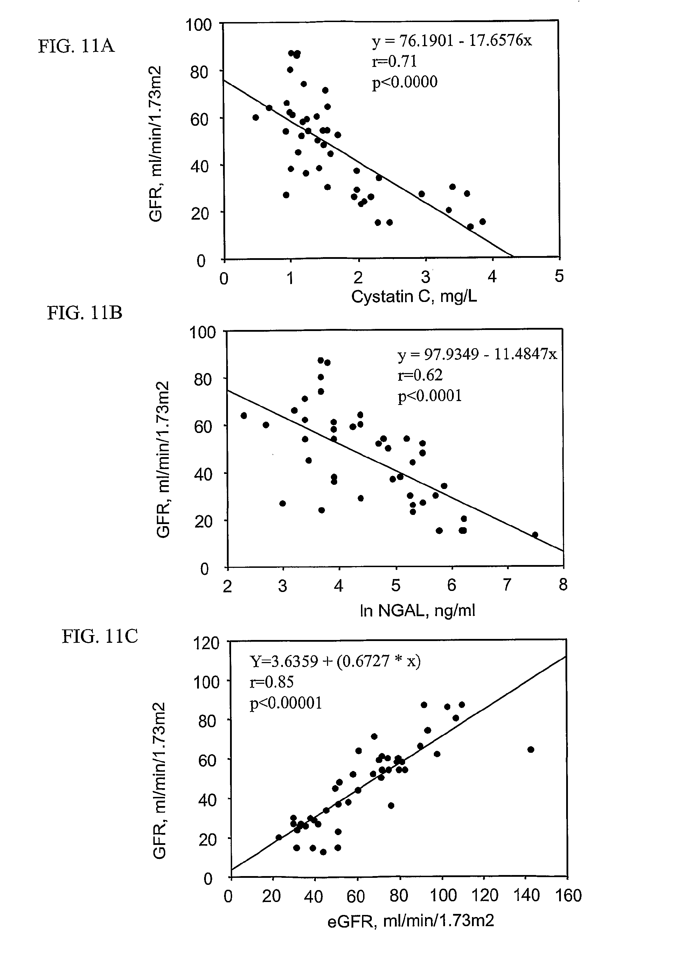

Neither NGAL nor cystatin C serum concentration had significant correlations with age, weight, height, sex, race or BMI (all *P*>0.1). However, serum NGAL and cystatin C levels were highly correlated (FIG. 9). In addition, both NGAL and cystatin C highly correlated with serum creatinine, eGFR (FIG. 10) and with measured GFR (FIG 11). Measured GFR was also highly correlated with eGFR (FIG. 11). The comparison of correlations of GFR with NGAL versus GFR with cystatin C was not statistically significant (Steiger's test, *P*=NS). Residual analyses were performed to evaluate the agreements between different predictor variables and measured GFR. The average percent difference from the predicted value was 31 ±4% for serum creatinine, 30 ±2.6% for cystatin C, 18 ±1.9% for eGFR, and 15 ±1.0 for NGAL. the following percentage of estimates were also detected with 30% of the predicted value of measured GFR: 89% of subjects for NGAL, 80% for eGFR, 66% for serum creatinine, and 58% for cystatin C.

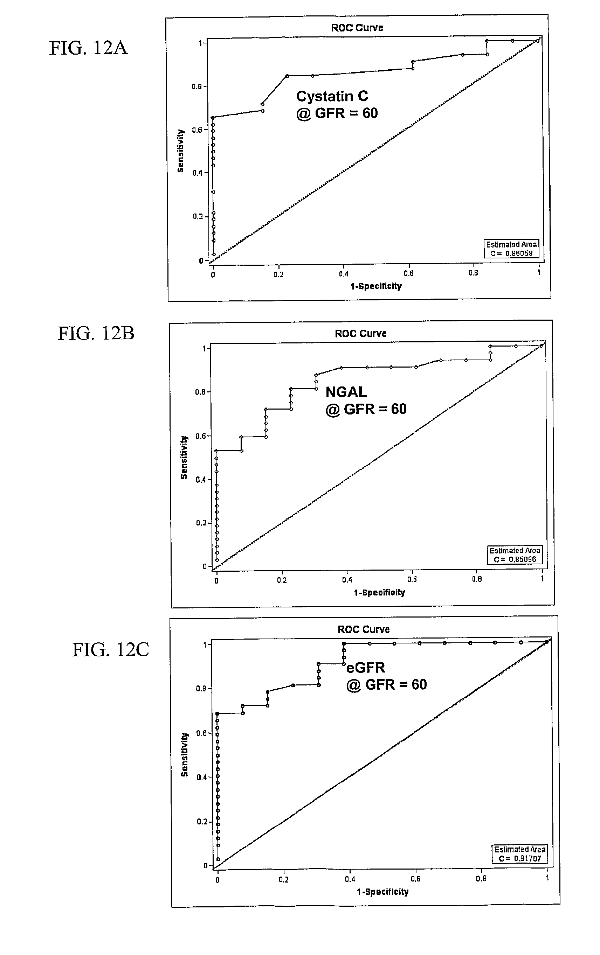

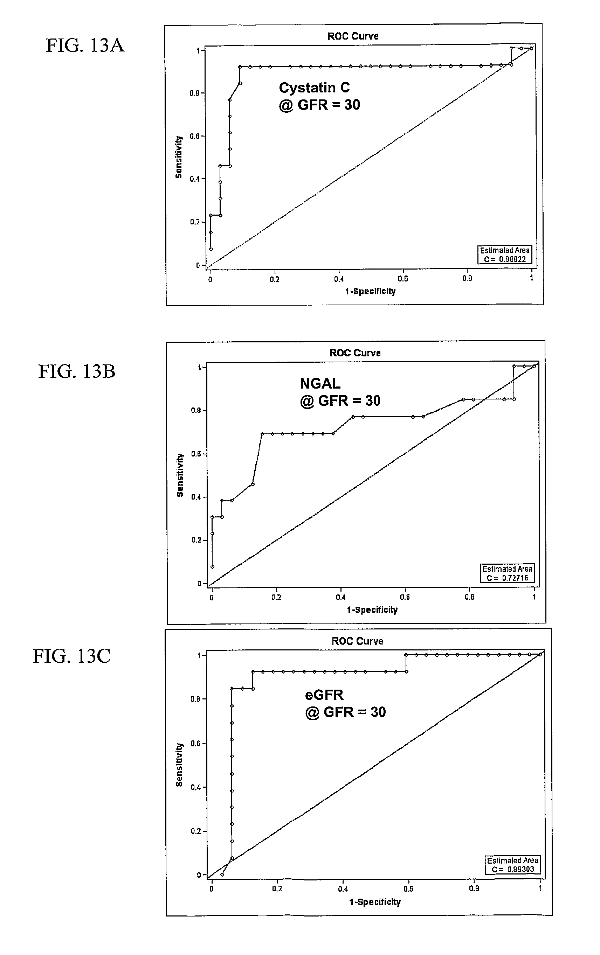

The Receiver Operating Characteristics (ROC) analyses are presented in FIGS. 12 and 13. For a cut-off point of GFR = 60 mL/min/1.73m², both serum NGAL, cystatin C and eGFR were all excellent biomarkers, with an AUC of 0.85, 0.86 and 0.92 respectively. For a cut-off point of GFR = 30 mL/min/1.73m², the diagnostic accuracy of cystatin C (AUC=0.89) was similar to that of eGFR (AUC=0.89) and slightly better than that of NGAL (AUC=0.73). The cut-off points for NGAL and cystatin C for the best diagnostic efficiencies at different GFR levels are shown in Table 7.

**TABLE 7**

| Variable | Sensitivity | Specificity | PPV | NPV |
|---|---|---|---|---|
| GFR 30 mL/min/1.73m² | | | | |
| Cystatin C =1.7 mg/L | 92 | 91 | 80 | 97 |
| NGAL = 190 ng/ml (In NGAL = 5.2 ng/ml) | 70 | 84 | 64 | 87 |
| | | | | |
| GFR=60 mL/min/1.73m² | | | | |
| Cystatin C =1.21 mg/L | 82 | 77 | 90 | 63 |
| NGAL = 45 ng/ml (In NGAL = 3.8 ng/ml) | 84 | 77 | 90 | 67 |

To further investigate the relationships between studied biomarkers and measured GFR, correlation analyses were performed at different CRD stages. For subjects with measured GFR ≥30 mL/min/1.73m² (n=30), there were significant correlations for all biomarkers tested (all *P*<0.0001), including cystatin C (r=0.45), NGAL (r=0.52), serum creatinine (r=0.70), and eGFR (r=0.72). However, for subjects with measured GFR <30 mL/min/1.73m² (n=15), NGAL was best correlated with measured GFR (r=0.62, *P*<0.0001), followed by cystatin C (r=0.41, *P*<0.0001). There was no significant correlation between measured GFR and either serum creatinine (r=0.12, *P*=0.66) or eGFR (r=0.20, *P*=0.47) at this advanced stage of CRD.

### b. Circulating NGAL correlates with other known biomarkers of CRD

This study of children with CRD demonstrated that (a) elevated levels of serum NGAL are characteristically present, (b) serum NGAL correlates closely with serum cystatin C, measured GFR, and eGFR, (c) both serum NGAL and cystatin C may prove useful in the quantitation of CRD, and (d) NGAL outperforms cystatin C and eGFR at lower levels of measured GFR.

The primary prerequisite for identification and staging of CRD is an exact measure of GFR. In this study, eGFR calculated using the Schwartz formula performed as well as cystatin C and NGAL in the overall correlation analyses and ROC analyses. However, while the ROC is a useful method for determining the sensitivity and specificity at specific cut-off values, it does not determine the individual variability of the parameter being studied. This was especially evident for eGFR as a marker in the lower ranges of measured GFR (higher level of kidney injury), at which both the measured serum creatinine and the eGFR performed poorly. These results are expected since it is well known that the Schwartz formula can overestimate kidney function in subjects with advanced kidney failure.

### (c) Circulating NGAL is the best overall biomarker for CRD

In our subjects, the best overall agreement with measured GFR was found for serum NGAL. While excellent agreement with measured GFR was evident for all biomarkers tested in patients with milder degrees of CRD, NGAL clearly outperformed cystatin C and eGFR at GFR levels of < 30 mL/min/1.73m² (at advanced degrees of CRD). Our results indicate that serum NGAL determination may provide an additional accurate measure of kidney dysfunction in CRD, especially in subjects with advanced CRD.

While the invention has been described in conjunction with preferred embodiments, one of ordinary skill after reading the foregoing specification will be able to effect various changes, substitutions of equivalents, and alterations to the subject matter set forth herein. Hence, the invention can be practiced in ways other than those specifically described herein. It is therefore intended that the protection herein be limited only by the appended claims and equivalents thereof.

All patents and publications recited herein are indicative of the levels of those skilled in the art to which the invention pertains. All patents and publications are herein incorporated by reference to the same extent as if each individual publication was specifically and individually indicated to be incorporated by reference.
The following embodiments of the invention are numbered as embodiments 1 to 28 and relate to:
1. A method for the early detection of a chronic renal injury in a mammal, comprising the steps of:
   (a) obtaining a urine sample from said mammal, wherein said mammal is not experiencing an acute renal injury;
   (b) determining the level of NGAL in said sample using an antibody of NGAL; and
   (c) evaluating the chronic renal injury status of said mammal, based on the level of NGAL in said urine sample.
2. The method of embodiment 1, wherein the mammal is a human.
3. The method of embodiment 1, wherein when the level of NGAL in the urine sample is less than a base cut-off level, the evaluated chronic renal injury status indicates normal kidney function.
4. The method of embodiment 3 wherein the base cut-off level is about 20 ng NGAL/mL of sample.
5. The method of embodiment 1, wherein when the level of NGAL in the urine sample is at or above an intermediate cut-off level, and up to an upper cut-off level, the evaluated chronic renal injury status indicates a mild chronic renal injury.
6. The method of embodiment 5, wherein the intermediate cut-off level is about 45 ng NGAL/mL of sample, and the upper cut-off level is about 150 ng NGAL/mL of sample.
7. The method of embodiment 1, wherein when the level of NGAL in the urine sample is at or greater than the upper cut-off level, the evaluated chronic renal injury status indicates advanced chronic renal injury.
8. The method of embodiment 7, wherein the upper cut-off level is about 150 ng NGAL/mL of sample.
9. The method of embodiment 1, wherein the evaluation of the chronic renal injury status of said mammal also includes (a) an assessment of the presence of an acute bacterial or viral infection or acute inflammation in said mammal, and (b) an assessment of the presence of an injury to another organ in said mammal.
10. The method of embodiment 1, wherein when said urine sample is obtained, said mammal is not experiencing another condition selected from the group consisting of an acute bacterial or viral infection, acute inflammation, injury to another organ, and cancer.
11. A method for the detection any change in a chronic renal injury status of a mammal, comprising the steps of:
   (a) obtaining a first urine sample from said mammalian, wherein said mammal is not experiencing an acute renal injury;
   (b) determining the level of NGAL in said first sample using an antibody for NGAL;
   (c) obtaining at least one subsequent urine sample after a period of time after obtaining the first urine sample;
      iv) determining the level of NGAL in said at least one subsequent urine sample using an antibody for NGAL; and
      v) evaluating the chronic renal injury status of said mammal, based on comparing the level of NGAL in said at least one subsequent urine sample to the level of NGAL in said first urine sample.
12. The method of embodiment 11, wherein a higher level of NGAL in said subsequent urine sample as compared to said first urine sample is an indication of worsening of the chronic renal injury in said mammal.
13. The method of embodiment 11, wherein a lower level of NGAL in said subsequent urine sample as compared to said first urine sample is an indication of improving of the chronic renal injury in said mammal.
14. The method of embodiment 11, wherein when said first urine sample and said at least one subsequent urine sample are obtained, said mammal is not experiencing another condition selected from the group consisting of an acute bacterial or viral infection, acute inflammation, injury to another organ, and cancer.
15. The method of embodiment 11, wherein said at least one subsequent urine sample comprises a plurality of subsequent urine samples obtained intermittently from said mammal.
16. The method of embodiment 11, wherein said mammal is a human.
17. A method of monitoring the effectiveness of a treatment for chronic renal injury in a mammal, comprising the steps of:
   (a) obtaining a baseline urine sample from a mammal, wherein said mammal is experiencing a chronic renal injury;
   (b) determining the level of NGAL in said baseline sample using an antibody of NGAL;
   (c) providing at least one treatment to said mammal for the chronic renal;
   (d) obtaining at least one post-treatment urine sample from said mammal;
   (e) determining the level of NGAL in said post-treatment urine sample using an antibody of NGAL; and
   (f) evaluating the effectiveness of the treatment, based on comparing the level of NGAL in said at least one post-treatment urine sample to the level of NGAL in said baseline urine sample.
18. The method of embodiment 17, wherein when said baseline urine sample and said at least one post-treatment urine sample are obtained, the mammal is not experiencing an acute renal injury.
19. The method of embodiment 17, wherein said at least one post-treatment urine sample comprises a plurality of post-treatment urine samples obtained intermittently from said mammal.
20. The method of embodiment 17, wherein said mammal is a human.
21. The method of embodiment 17, wherein a higher level of NGAL in said post-treatment urine sample as compared to said baseline urine sample is an indication of worsening of the chronic renal injury in said mammal.
22. The method of embodiment 11, wherein a lower level of NGAL in said post-treatment urine sample as compared to said baseline urine sample is an indication of improving of the chronic renal injury in said mammal.
23. The method of embodiment 17, wherein the at least one post-treatment urine sample is obtained after a period of time following the time of the treatment.
24. The method of embodiment 17, further comprising the steps of:
   (g) providing one or more additional post-treatment urine samples;
   (h) determining the level of NGAL in said one or more additional post-treatment urine samples using an antibody for NGAL; and
      ix) evaluating the effectiveness of the treatment, based on comparing the level of NGAL in the one or more additional post-treatment urine sample, to the level of NGAL in the baseline urine sample or in a preceding post-treatment urine sample.
25. The method of embodiment 24, wherein the step of providing treatment is continued until the level of NGAL in the one or more additional post-treatment urine sample is either no longer increased or has decreased.
26. The method of embodiment 17, wherein the chronic renal injury is selected from the group consisting of chronic infections, chronic inflammation, glomerulonephritides, vascular diseases, interstitial nephritis, drugs, toxins, trauma, renal stones, long standing hypertension, diabetes, congestive heart failure, nephropathy from sickle cell anemia and other blood dyscrasias, nephropathy related to hepatitis, HIV, parvovirus and BK virus, cystic kidney diseases, congenital malformations, obstruction, malignancy, kidney disease of indeterminate causes, lupus nephritis, membranous glomerulonephritis, membranoproliferative glomerulonephritis, focal glomerular sclerosis, minimal change disease, cryoglobulinemia, ANCA-positive vasculitis, ANCA-negative vasculitis, amyloidosis, multiple myeloma, light chain deposition disease, complications of kidney transplant, chronic rejection of a kidney transplant, chronic allograft nephropathy, and the chronic effects of immunosuppressives.
27. A method of identifying the extent of chronic renal injury in a mammal over time, comprising the steps of:
   (a) obtaining at least one first urine sample at a first time from said mammal, wherein said mammal is not experiencing an acute renal injury;
   (b) determining the level of NGAL in said first urine sample using an antibody for NGAL;
   (c) obtaining at least one subsequent urine sample obtained at a time which is subsequent to said first time from said mammal;
   (d) determining the level of NGAL in said at least one subsequent urine sample using an antibody for NGAL; and (e) determining the extent of the chronic renal injury in said mammal over time, based on comparing the level of NGAL in said at least one subsequent urine sample to the level of NGAL in said first urine sample.
28. The method of embodiment 27, wherein said at least one subsequent urine sample is obtained after a period of time from obtaining said first urine sample.

## Claims

1. A method for evaluating the chronic renal injury status in a mammal, comprising the steps of:
(a) determining the level of NGAL in a sample of urine obtained from a mammal, wherein said mammal has been determined not to be experiencing an acute renal injury, using an antibody for NGAL; and
(b) evaluating the chronic renal injury status of said mammal, based on the level of NGAL in said urine sample.

2. The method according to Claim 1, wherein the mammal is a human.

3. The method according to Claim 1, wherein when the level of NGAL in the urine sample is less than a base cut-off level of about 20 ng NGAL/mL of sample, the evaluated chronic renal injury status indicates normal kidney function.

4. The method according to Claim 1, wherein when the level of NGAL in the urine sample is at or above an intermediate cut-off level of about 45 ng NGAL/mL of sample and up to an upper cut-off level of about 150 ng NGAL/mL of sample, the evaluated chronic renal injury status indicates a mild chronic renal injury.

5. The method according to Claim 1, wherein when the level of NGAL in the urine sample is at or greater than the upper cut-off level of about 150 ng NGAL/mL of sample, the evaluated chronic renal injury status indicates advanced chronic renal injury.

6. The method according to any of the preceding Claims, used for the detection of any change in a chronic renal injury status of a mammal, wherein the sample evaluated in Claim 1 is a first urine sample, further comprising the steps of:
(c) determining the level of NGAL in at least one subsequent urine sample obtained from said mammal after a period of time of at least one week after the first urine sample was obtained, and
(d) detecting any change in the chronic renal injury status of said mammal, based on comparing the level of NGAL in said at least one subsequent urine sample to the level of NGAL in said first urine sample.

7. The method according to Claim 6, wherein a higher level of NGAL in said subsequent urine sample as compared to said first urine sample is an indication of worsening of the chronic renal injury in said mammal, and wherein a lower level of NGAL in said subsequent urine sample as compared to said first urine sample is an indication of improving of the chronic renal injury in said mammal.

8. The method of Claim 6 or 7, used for monitoring the effectiveness of a treatment for chronic renal injury in a mammal, wherein the first urine sample is a baseline urine sample and the at least one subsequent urine sample is a post-treatment urine sample and the effectiveness of the treatment is evaluated by comparing the level of NGAL in the post-treatment urine sample to the level of NGAL in the baseline urine sample.

9. The method according to Claim 8, wherein a lower level of NGAL in said post- treatment urine sample as compared to said baseline urine sample is an indication that the treatment was effective for improving of the chronic renal injury in said mammal.

10. The method according to any of the preceding Claims, wherein the chronic renal injury includes or is caused by chronic infections, chronic inflammation,
glomerulonephritides, vascular diseases, interstitial nephritis, drugs, toxins, trauma, renal stones, long standing hypertension, diabetes, congestive heart failure, nephropathy from sickle cell anemia and other blood dyscrasias, nephropathy related to hepatitis, HIV, parvovirus and BK virus, cystic kidney diseases, congenital malformations, obstruction, malignancy, kidney disease of indeterminate causes, lupus nephritis, membranous glomerulonephritis, membranoproliferative glomerulonephritis, focal glomerular sclerosis, minimal change disease, cryoglobulinemia, ANCA-positive vasculitis, ANCA-negative vasculitis, amyloidosis, multiple myeloma, light chain deposition disease, complications of kidney transplant, chronic rejection of a kidney transplant, chronic allograft nephropathy, and the chronic effects of immunosuppressives.
